# EUROPEAN PATENT SPECIFICATION

(11) **EP 3 510 992 B1**
(45) Date of publication and mention of the grant of the patent: **17.02.2021**
(21) Application number: 17849011.6
(22) Date of filing: 28.08.2017
(51) Int. Cl.: A61K 8/60, A61K 8/02, A61Q 19/02, A61K 8/99

(54) **SKIN WHITENING COMPOSITION CONTAINING MANNOSYLERYTHRITOL LIPID**
HAUTAUFHELLENDE ZUSAMMENSETZUNG MIT MANNOSYLERYTHRITOLLIPID
COMPOSITION DE BLANCHIMENT DE LA PEAU CONTENANT UN LIPIDE MANNOSYLÉRYTHRITOL

(30) Priority: 08.09.2016 KR 20160115380
(43) Date of publication of application: 17.07.2019
(73) Proprietor: Amorepacific Corporation, Seoul 04386 (KR)
(72) Inventor: YOO, Jae Won, Yongin-si Gyeonggi-do 17074 (KR); HWANG, Yoon Kyun, Yongin-si Gyeonggi-do 17074 (KR); BIN, Sung-Ah, Yongin-si Gyeonggi-do 17074 (KR); KIM, Yong Jin, Yongin-si Gyeonggi-do 17074 (KR); LEE, John Hwan, Yongin-si Gyeonggi-do 17074 (KR)
(74) Representative: dompatent von Kreisler Selting Werner - Partnerschaft von Patent- und Rechtsanwälten mbB
(86) International application number: PCT/KR2017/009369
(87) International publication number: WO 2018/048127

(56) References cited:
- EP-A1- 2 578 204
- WO-A1-2007/060956
- JP-A- 2012 140 388
- KR-A- 20160 000 747
- DATABASE GNPD [Online] MINTEL; 1 January 2015 (2015-01-01), LG Household: "Essential Skin Toner", XP002792259, Database accession no. 2824471
- DATABASE WPI Week 200949 Thomson Scientific, London, GB; AN 2009-L30677 XP002792260, & JP 2009 149566 A (TOYOBO KK) 9 July 2009 (2009-07-09)
- DATABASE WPI Week 200981 Thomson Scientific, London, GB; AN 2009-R57644 XP002792261, & JP 2009 275017 A (TOYOBO KK) 26 November 2009 (2009-11-26)
- DATABASE WPI Week 201112 Thomson Scientific, London, GB; AN 2011-A54619 XP002792262, & JP 2011 001312 A (TOYOBO KK) 6 January 2011 (2011-01-06)
- ZHAO, XIAOXIAN et al.: "Mannosylerythritol Lipid Is a Potent Inducer of Apoptosis and Differentiation of Mouse Melanoma Cells in Culture", Cancer Research, vol. 59, no. 2, 15 January 1999 (1999-01-15), pages 482-486, XP055139167,
- FAN, LINLIN et al.: "Characterization and Inducing Melanoma Cell Apoptosis Activity of Mannosylerythritol Lipids-A Produced from Pseudozyma Aphidis", PLoS ONE, vol. 11, no. 2, 1 February 2016 (2016-02-01), pages 1-17, XP055497462, DOI: doi:10.1371/journal.pone.0148198
- TAKAHASHI, MAKOTO et al.: "Glycolipid Biosurfactants, Mannosylerythritol Lipids, Show Antioxidant and Protective Effects against H202-induced Oxidative Stress in Cultured Human Skin Fibroblasts", Journal of Oleo Science, vol. 61, no. 8, 2012, pages 457-464, XP055497467, DOI: doi:10.5650/jos.61.457

## Description

### [Technical Field]

The present invention relates to a skin whitening composition containing mannosylerythritol lipid (MEL) as an effective ingredient.

### [Background Art]

The skin is equipped with physical and chemical UV protection factors and has a mechanism to minimally prevent the skin damage caused by various photochemical reactions. The stratum corneum of the skin attenuates the energy of the ultraviolet ray by reflecting and diffusing the ultraviolet ray, and also the melanin pigment, the superoxide dismutase (SOD), other antioxidant ingredients and the like absorb the ultraviolet ray penetrated into the inside of the skin and attenuate its energy or protect the skin from damage by clearing active oxygen secondarily generated by the ultraviolet ray.

However, when a large amount of ultraviolet ray exceeding the ability of the defensive factor as described above is received by the body or its ability is deteriorated with age, various skin damages occur.

There are various cells in the skin, which are responsible for immunity in the body system, such as macrophages and langerhans cells. These cells are not only reduced numerically but also functionally impaired by ultraviolet ray irradiation. Among the factors that determine skin color, the biggest contributing factor is the distribution and amount of melanin in the skin. The melanin is produced in cells called as melanocytes, and these melanocytes contain enzymes such as tyrosinase and the like, and these enzymes act together, thereby inducing the polymerization oxidation reaction with an amino acid, called as tyrosine, as a substrate, which is always present in the body, to form melanin, a dark brown pigment. The melanin thus formed migrates to the epidermal cell, called as keratinocyte, through the dendrites of melanocytes. Here, the melanin forms a hat-like structure around the nucleus, thereby playing important roles, such as protecting genes from ultraviolet rays, removing free radicals, and protecting the intracellular proteins.

These enzymes that degrade the melanin are not present in the body, but when the keratinocyte ages, exhausts its function, and falls off the epidermis, the melanin is removed from the skin together. However, when the melanin is largely produced more than necessary, hyperpigmentation such as melasma or freckle, spot or the like are induced, thereby resulting in poor cosmetic results.

Several factors affecting melanin production have been known in the art, and the hyperactivity of melanin production caused by ultraviolet ray and the subsequent pigmentation is very important in the field of cosmetic product. The basic mechanisms of pharmaceuticals incorporated into whitening cosmetic products for the prevention of pigmentation are inhibition of the tyrosinase action, inhibition of the tyrosinase production, inhibition of the melanin producing intermediate, inhibition of the reduction and photo-oxidation of the existing melanin, promotion of the melanin excretion, ultraviolet ray cut and the like.

According to desire to have a white skin even in the ultraviolet ray exposure environment, there is a growing need for prevention and improvement of skin dyschromatosis and hyperpigmentation. Therefore, it has become necessary to develop whitening products that inhibit excessive melanin production, and many efforts have been made in that time. Specific examples thereof include inhibitors, which inhibit tyrosinase activity, such as kojic acid and arbutin, hydroquinone, vitamin A, vitamin C and derivatives thereof. However, they are limited in their use due to insufficient whitening effect, and problems of stability within the formulation and safety to the skin. In these regards, studies on raw materials derived from natural materials, which are highly effective for skin whitening and also are easily formulated and are safe for skin, are actively being carried out.

### [Prior Art Document]

### [Patent Document]

Korean Patent Registration No. 1225267, "BIOSURFACTANT-CONTAINING SKIN CARE COSMETIC AND SKIN ROUGHNESS-IMPROVING AGENT".

### [Disclosure]

### [Technical Problem]

As described above, although many existing whitening-effect ingredients have been reported, it is difficult to apply a sufficient amount to the formulation to achieve a whitening effect due to low solubility, or even when a sufficient amount is applied, the use of additional ingredients is necessary to formulate, and thus there were many cases where there was limitation in the application to the formulation or influence on the feeling of use of the formulation. Accordingly, there is a high need for ingredients that are easy to formulate and have whitening effect, without using additional ingredients for formulation. Accordingly, the inventors of the present invention have conducted research on a biosurfactant obtained from the fermentation of microorganisms, and as a result, have confirmed that mannosylerythritol lipid (MEL) shows excellent efficacy in whitening and is very easy to formulate without using additional ingredients, and thus completed the present invention.

Therefore, it is an object of the present invention to provide a skin whitening composition that is easily formulated while having high skin whitening efficacy and is safe for skin.

### [Technical Solution]

In order to accomplish the above object, the present invention provides a skin whitening composition containing mannosylerythritol lipid (MEL) as an effective ingredient. At this time, the mannosylerythritol lipid may be a mannosylerythritol lipid hydrogenated through hydrogenation.

### [Advantageous Effects]

The skin whitening composition containing MEL according to the present invention inhibits the formation of skin melanocytes, and improves the overall skin tone, and thus gives effectiveness of exhibiting clean, darkness-relieved, and bright skin, thereby imparting whitening efficacy. In addition, since MEL itself is a biosurfactant, it is easy to formulate and it can be applied to a wide variety of formulations with high content without the use of specific additional ingredients to formulate the potency ingredients.

### [Description of Drawings]

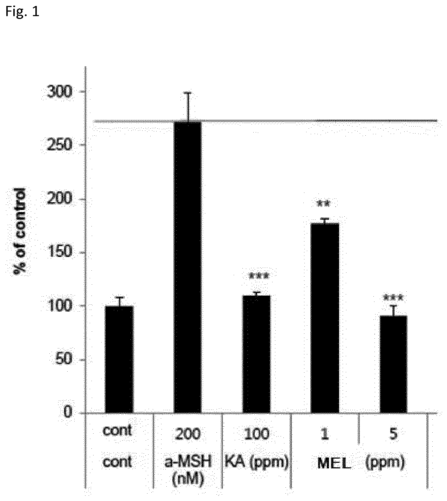

FIG. 1 is data showing the melanin production inhibitory effect of MEL according to the present invention.

### [Best Mode]

Hereinafter, the present invention will be described in detail.

The mannosylerythritol lipid (hereinafter referred to as "MEL") used in the present invention is a glycolipid composed of mannose, sugar alcohol and fatty acid. The MEL, known as a biosurfactant, is a glycolipid-based substance formed by binding a fatty acid to a sugar called as mannosylerythritol and has a minimum surface tension of 29 dyne/cm and a critical micelle concentration (CMC) of 15 µM (10 mg/ℓ) and exhibits surface activity almost similar to that of a chemically synthesized surfactant used in the past. In addition, the MEL exhibits high ability to lower interfacial tension, high emulsification ability, pH and thermal stability and the like. Since the MEL having such characteristics easily forms a lamellar structure, it can be easily formulated.

The MEL used in the present invention can be obtained by fermenting a vegetable oil with a microorganism. Examples of usable microorganisms may include *Candida sp., Torulopsis sp., Pseudomonas sp., Bacillus sp., Alcaligenes sp., Acinetobacter sp., Ustilago sp., Rhodococcus sp.* and the like.

In addition, as a skin whitening composition of the present invention, the MEL is represented by the following formula 1: 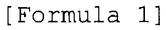 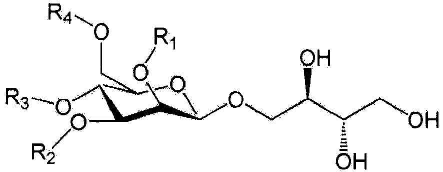 (wherein R₁ and R₂ are the same or different from each other and are each independently a C2 to C24 aliphatic acyl group, and R₃ and R₄ are the same or different from each other and are each independently an acetyl group or hydrogen.).

More preferably, R₁ and R₂ are the same or different from each other and each independently can be a C6 to C18 aliphatic acyl group.

In formula 1, if R₁ = R₂ = Ac and R₃ = R₄ = Ac, it is classified as MEL-A, if R₁ = R₂ = Ac, R₃ = H, and R₄ = Ac, it is classified as MEL-B, if R₁ = R₂ = Ac, R₃ = Ac, and R₄ = H, it is classified as MEL-C, if R₁ = R₂ = Ac, R₃ = H, and R₄ = H, it is classified as MEL-D. The MEL applicable to the present invention may be any one of MEL-A, MEL-B, MEL-C, or MEL-D alone, but two or more MELs may be used in combination.

In the present invention, the preparation method of the MELs is not particularly limited, but advantageously, a fermentation method using microorganisms is arbitrarily selected. For example, the MELs (MEL-A, MEL-B, MEL-C, and MEL-D) can be cultured by Pseudozyma antarctica NBRC 10736 according to a conventional method, and examples of microorganisms may include Pseudozyma antarctica, Pseudozyma sp. and the like. It is a well-known fact that the mixture of the MELs is readily obtained from any microorganism. The mixture of the MELs can be separated or purified in high purity into MEL-A, MEL-B, MEL-C and MEL-D through various purification methods. The microorganisms having the ability to produce the MEL are not particularly limited and can be suitably used according to the purpose.

In addition, the MELs obtained by hydrogenating an unsaturated bond in -(CH₂)ₙ- by adding hydrogen to the MELs can be used as a skin whitening composition. These hydrogenated MELs have the effect of preventing oxidization and inhibiting disodoration when the ingredient is stabilized and formulated. More specifically, the hydrogenation reaction is carried out by dissolving MEL alone or in an appropriate organic solvent, and then using a catalyst and hydrogen. The organic solvent used herein may be various organic solvents, and preferably may be ethyl acetate, ethanol and the like. The catalyst used herein may be various catalysts used for the hydrogenation reaction and preferably may be Pd/C, PtO₂ and the like. The hydrogen used here can be reacted under a pressure of 1 to 100 atm, preferably 1 to 5 atm.

Although the amount of MEL added in the skin whitening composition varies depending on the kind of the cosmetics or external preparations to be used and thus cannot be uniformly defined, with respect to various cosmetics or external preparations, it is preferably 0.001 to 50 wt.%, more preferably 0.01 to 20 wt.%, and particularly preferably 0.05 to 5 wt.%. Here, the mode of use of the MEL added to the cosmetics or external preparations is arbitrary. For example, the MEL can be used as an extract, as it is, from a culture medium or as a purified high purity substance, or can be suspended in water or can be used in the form of a solution dissolved in polyol, oil or the like.

The skin whitening composition containing MEL according to the present invention is preferably a cosmetic composition or a composition of the external preparation for skin, which may further include at least one selected from the group consisting of polyphenol derivatives, kojic acid and derivatives thereof, niacinamide and 3,4,5-trimethoxyphenyl-based ester compound in an amount of 0.1 to 3.0 wt.% based on the total weight of the composition. These substances are ingredients that have proven to have whitening effect through mechanisms such as inhibiting the activity of tyrosinase or obstructing the transfer of melanin from melanocytes to keratinocytes.

The MEL proposed in the present invention may also be incorporated into cosmetics by dissolving it in a polyol-based oil-soluble base, or an oil-soluble ingredient, and can be incorporated into cosmetics in the form of liposomes, especially to water-based cosmetics such as toilet water, moisturizing liquid and the like.

In addition, the cosmetics of the present invention may further include conventionally known surfactants, preservatives, fragrances, moisturizing agents, salts, solvents, resins, antioxidants, chelating agents, neutralizing agents, pH adjusting agents, insect repellents and physiologically active ingredients within the range not impairing the purpose of the present invention.

The surfactant is added separately from MEL and is a material that helps to solubilize MEL, and preferably may be, but is not limited to, at least one selected from polyoxyethylene hydrogenated castor oil, ceteareth, ceteth, glycereth, laureth, oleth, polysorbate, steareth, amino acid-fatty acid ester, polyoxyethylene stearate.

The cosmetic composition for skin whitening according to the present invention is not particularly limited in its formulation. For example, the cosmetic composition for skin whitening may be formulated as emollient toilet water, astringent toilet water, nourishing toilet water, nourishing creams, massage creams, essence, eye creams, eye essence, cleansing creams, cleansing foams, cleansing water, packs, powders, body lotions, body creams, body oils, body essence, makeup base, foundations or the like, and also may be formulated as cosmetics for skin whitening or as medicinal products such as ointments and patches.

In addition, the MEL proposed in the present invention can be applied to composition of the external preparation for skin. When the skin whitening composition is used as a composition of the external preparation for skin, it may be formulated while containing a carrier, medium or base acceptable in the field of dermatology. In addition, the skin whitening composition may contain adjuvants conventionally used in the field of dermatology, such as fatty substances, organic solvents, solubilizers, thickening and gelling agents, softening agents, antioxidants, suspending agents, stabilizers, foaming agents, fragrances, surfactants, water, ionic or non-ionic emulsifiers, fillers, chelating agents, preservatives, vitamins, blocking agents, wetting agents, essential oils, dyes, pigments, hydrophilic active agents, lipophilic active agents, lipid vesicles or any other ingredients normally used in external preparations for skin. In addition, these ingredients can be introduced in amounts commonly used in the field of dermatology.

The composition of the external preparation for skin may be, but is not limited to, a formulation selected from the group consisting of ointments, pastes, lotions, creams, gels, solutions, suspensions, emulsions, patches and sprays.

In the composition of the external preparation for skin, the effective amount of the skin antioxidant composition according to the present invention may vary depending on the constituents of the composition of the external preparation for skin, the type of the formulation, and the age, weight, health condition and sex of the user, administration time, route of administration and administration method. For example, the content of the skin antioxidant composition of the present invention may be 0.0001 to 10 wt.%, preferably 0.0001 to 1 wt.%, based on 100 wt.% of the total composition of the external preparation for skin. If the skin antioxidant composition of the present invention is contained in an amount of less than 0.0001 wt.%, sufficient antioxidative effect cannot be expected. If the skin antioxidant composition is contained in an amount exceeding 10 wt.%, side effects such as itching, urticaria and allergies may occur.

Hereinafter, the present invention will be described in more detail with reference to embodiments.

The mannosylerythritol lipids (MELs) used in the present invention were purchased from an external supplier, and the composition of MELs comprises MEL-B in an amount of about 90% or more.

### Experimental Example 1: Confirmation of whitening effect

The inhibitory effect on melanin production in melanin pigment producing cells by the mannosylerythritol lipid (hereinafter referred to as MEL) was measured. As the cell line and serum, the mouse-derived B16F10 (melanoma cell) cell line purchased from ATCC and FBS (Cat No. 30-2020) were used. The DMEM (Cat No. 11995) required for cell culture and antibiotic-antifungal agent (Cat No. 15240-062) were purchased from Invitrogen company (GIBCO). The cell line was cultured under the conditions of 37°C, 10% CO₂. The cultured B16F10 cells were removed with 0.05% trypsin-EDTA and inoculated again into the 48-well plate at the same number (1 x 10⁴ cells/well), and then for three consecutive days from the second day, the medium containing α-MSH (200 mM) was replaced with phenol-free DMEM medium to be treated with 1 or 5 ppm of MEL. Kojic acid was used as a positive control. After the fifth day, 1N NaOH was added and reacted at 60 °C for 2 hours to dissolve the melanin contained in the cells, and then measured the amount of melanin by measuring the absorbance at 405 nm.

As shown in FIG., when the expression level of melanin pigment in B16F10 cells cultured on the untreated medium was regarded as 100%, the amount of melanin pigment expressed by B16F10 cells in a medium supplemented with 200 nM melanocyte stimulating hormone (α-MSH) was about 270%. It was confirmed that the expression amount of melanin pigment by B16F10 cells in the medium supplemented with α-MSH and 100 ppm of kojic acid is about 110%, and thus the expression of melanin pigment was inhibited, and it was confirmed that the expression amount of melanin pigment in the medium supplemented with α-MSH and 1 ppm of MEL was about 180%, and the expression amount of melanin pigment in the medium supplemented with α-MSH and 5 ppm of MEL was about 90% and thus the expression of melanin pigment was inhibited. It was confirmed that the MEL exhibits whitening efficacy at very low concentrations below 1/20 of kojic acid.

### Experimental Example 2: Confirmation of compatibility with solvent

1% of the MEL was added to major ingredients commonly used in common cosmetic formulations and stirred, and then the presence or absence of precipitation or separation at room temperature over time was visually observed and shown in Table 1 below.

**Table 1:**

| | Compatibility |
|---|---|
| Water | Δ |
| 1, 3-BG | ○ |
| Eutanol-G | ○ |
| CEH | ○ |
| CSA | ○ |

| | |
|---|---|
| (o : transparent solution, Δ : opaque solution, X : separation or precipitation) | |

In Table 1, 1,3-BG is 1,3-butylene glycol, Eutanol-G is octyldodecanol, CEH is cetyl ethylhexanoate, and CSA is caprylic-capric triglyceride.

### Experimental Example 3: Confirmation of improvement of odor of hydrogenated MEL raw material

After dissolving MEL and hydrogenated MEL in an appropriate solvent at a concentration of 0.5%, specific odor at room temperature, sensitivity evaluation for disodoration when heating in a water bath for 3 hours, and disodoration under the daylight storage condition were carried out, and as a result, it was confirmed that the disodoration of MEL was improved by hydrogenation as shown in Table 2 below.

**Table 2:**

| | Room temperature | Heating | Daylight |
|---|---|---|---|
| MEL | ○ | Δ | Δ |
| Hydrogenated MEL | ○ | ○ | ○ |

| | | | |
|---|---|---|---|
| (o: good, Δ : slightly specific odor or disodoration, X : serious specific odor or disodoration) | | | |

### Formulation Example 1: Preparation of nourishing cream

A nourishing cream containing the skin whitening composition of the present invention was prepared in a conventional manner according to the composition shown in Table 3 below.

**Table 3:**

| Ingredient | Nourishing cream (unit: wt.%) |
|---|---|
| MEL | 1.0 |
| α-ketoglutaric acid | 1.0 |
| Niacinamide | 1.0 |
| hydrogenated vegetable oil | 1.5 |
| Stearic acid | 0.6 |
| Glycerol stearate | 1.0 |
| Stearyl alcohol | 2.0 |
| Polyglyceryl-10 pentastearate & Behenyl alcohol & Sodium stearoyl lactylate | 1.0 |
| Arachidyl behenyl alcohol & Arachidylglucoside | 1.0 |
| Cetearyl alcohol & Cetearylglucoside | 2.0 |
| PEG-100 stearate & Glycerololeate & Propylene glycol | 1.5 |
| Caprylic/caprlic triglyceride | 11.0 |
| Cyclomethicone | 6.0 |
| Preservative, Fragrance | 0.1 |
| Triethanolamine | 0.1 |
| Purified water | Remainder |

### Formulation Example 2: Preparation of nourishing toilet water

Nourishing toilet water containing the skin whitening composition of the present invention was prepared in a conventional manner according to the composition shown in Table 4 below.

**Table 4:**

| Ingredient | Nourishing toilet water (unit: wt.%) |
|---|---|
| MEL | 1.0 |
| α-Ketoglutaric acid | 1.0 |
| Niacinamide | 1.0 |
| β-1,3-Glucan | 1.0 |
| Beeswax | 4.0 |
| Polysorbate | 1.5 |
| Sorbitan sesquioleate | 1.5 |
| Liquid paraffin | 0.5 |
| Caprylic/Caprlic triglyceride | 5.0 |
| Glycerin | 3.0 |
| Butylene glycol | 3.0 |
| Propylene glycol | 3.0 |
| Carboxyvinyl polymer | 0.1 |
| Triethanolamine | 0.2 |
| Preservative, Fragrance | 0.1 |
| Purified water | Remainder |

### Formulation Example 3: Preparation of ointment

An ointment containing the skin whitening composition of the present invention was prepared in a conventional manner according to the composition of Table 5 below.

**Table 5:**

| Ingredient | Ointment (unit: wt.%) |
|---|---|
| MEL | 1.0 |
| α-ketoglutaric acid | 1.0 |
| Niacinamide | 1.0 |
| β-1,3-Glucan | 10.0 |
| Beeswax | 10.0 |
| Polysorbate | 5.0 |
| PEG-60 hydrogenated caster oil | 2.0 |
| Sorbitan sesquioleate | 0.5 |
| Vaseline | 5.0 |
| Liquid paraffin | 10.0 |
| Squalane | 5.0 |
| Shea butter | 3.0 |
| Caprylic/Caprlic triglyceride | 5.0 |
| Glycerin | 10.0 |
| Propylene glycol | 10.2 |
| Triethanolamine | 0.2 |
| Preservative, Fragrance | 0.1 |
| Purified water | Remainder |

### Formulation Example 4: Preparation of gel

A gel containing the skin whitening composition of the present invention was prepared in a conventional manner according to the composition of Table 6 below.

**Table 6:**

| Ingredient | Gel (unit: wt.%) |
|---|---|
| MEL | 1.0 |
| α-Ketoglutaric acid | 1.0 |
| Niacinamide | 1.0 |
| β-1, 3-Glucan | 0.1 |
| Ethylenediamine sodium acetate | 0.05 |
| Glycerin | 5.0 |
| Carboxyvinyl polymer | 0.3 |
| Ethanol | 5.0 |
| PEG-60 hydrogenated caster oil | 0.5 |
| Triethanolamine | 0.3 |
| Preservative, Fragrance | 0.1 |
| Purified water | Remainder |

## Claims

1. Use of mannosylerythritol lipid (MEL) as a skin whitening agent.

2. The use according to claim 1, wherein the mannosylerythritol lipid is used in a skin whitening composition in an amount of 0.01 to 20 wt.% based on 100 wt.% of the total composition.

3. The use according to claim 1, wherein the mannosylerythritol lipid is one in which the unsaturated bond contained in the aliphatic acyl group is hydrogenated.

4. A cosmetic composition suitable for skin whitening, wherein the composition comprises mannosylerythritol lipid in which the unsaturated bond contained in the aliphatic acyl group is hydrogenated.

## Patentansprüche

1. Verwendung von Mannosylerythrit-Lipid (MEL) als Hautaufhellungsmittel.

2. Verwendung gemäß Anspruch 1, wobei das Mannosylerythrit-Lipid in einer Hautaufhellungszusammensetzung in einer Menge von 0,01 bis 20 Gew.-% verwendet wird, bezogen auf 100 Gew.-% der Gesamtzusammensetzung.

3. Verwendung gemäß Anspruch 1, wobei das Mannosylerythrit-Lipid eines ist, bei dem die in der aliphatischen Acylgruppe enthaltene ungesättigte Bindung hydriert ist.

4. Kosmetische Zusammensetzung, die für die Hautaufhellung geeignet ist, wobei die Zusammensetzung Mannosylerythrit-Lipid umfasst, bei dem die in der aliphatischen Acylgruppe enthaltene ungesättigte Bindung hydriert ist.

## Revendications

1. Utilisation d'un lipide de mannosylérythritol (MEL) en tant qu'agent d'éclaircissement de la peau.

2. Utilisation selon la revendication 1, dans laquelle ledit lipide de mannosylérythritol est utilisé dans une composition d'éclaircissement de la peau en une quantité de 0,01 à 20 % en poids, par rapport à 100 % en poids de la composition totale.

3. Utilisation selon la revendication 1, dans laquelle ledit lipide de mannosylérythritol est tel que la liaison insaturée contenue dans le groupe d'acyle aliphatique est hydrogéné.

4. Composition cosmétique appropriée pour l'éclaircissement de la peau, dans laquelle ladite composition comprend un lipide de mannosylérythritol où la liaison insaturée contenue dans le groupe d'acyle aliphatique est hydrogéné.
